# EUROPEAN PATENT APPLICATION

(11) **EP 3 882 359 A1**
(43) Date of publication of application: **22.09.2021**
(21) Application number: 20164295.6
(22) Date of filing: 19.03.2020
(51) Int. Cl.: C12Q 1/6809, C12Q 1/6869, G01N 33/50

(54) **SINGLE CELL RNA SEQUENCING**

(71) Applicant: Singleron Biotechnologies GmbH, 51105 Köln (DE)
(72) Inventor: FANG, Nan, 41468 Neuss (DE); FAN, Jue, Shanghai (CN); CHEN, Yunmei, Nanjing, Jiangsu (CN); GAO, Yue, Nanjing, Jiangsu (CN)
(74) Representative: CH Kilger Anwaltspartnerschaft mbB

(57) **Abstract**

The present invention refers to scRNA-seq for high throughput drug screening and repurposing with primary cells or cell lines. It refers to a method for assaying a cell-based drug screening comprising the steps of: culturing a cell population in a culturing environment; exposing the cell population to drug substances, wherein defined monitoring points in time and concentration of the drug substances are set; generating a single cell library after exposure to drug substances; performing single cell RNA sequencing of the single cell libraries on a plurality of genes of the individually sorted cells from the cell library; and performing bioinformatic analysis to identify differentially expressed genes. Also, it refers to the use of a method for assaying a cell-based drug screening, wherein differentially expressed genes are potential marker genes and are used to screen drugs for cancers.

## Description

### Field of the Invention

The present invention is in the field of molecular biology and drug development. In particular, the invention relates to mammalian cell culture, single cell RNA sequencing (scRNA-seq) and drug screening.

### Background of the invention

The drug screening could be derived from transcriptomic (RNA), proteomic and metabolomic data. Before and after treatment with a drug, the transcriptomic signature is derived by comparing the gene expression profile of cells or tissues. Drugs developed by prior bulk methods encounter problems, in which drugs that work for some patients may have little or even no effect for other patients, or a drug may lose its efficacy gradually during treatment in some patients, although it is seemingly effective at the beginning of treatment. This is partially due to the heterogeneity at different levels, so as to say, the expression profiles among different individuals, or different tumors within one individual, as well as different cells within one tumor, may vary and thus respond differently to a drug. As a result, the drug may only work on some but not all tumor cells (denoted herein as subgroup), leaving other subgroups to survive and recover into prosperity when the treatment is ceased. There is also possibility that new subgroups of tumor cells might even evolve during the treatment with certain drugs. This causes plenty of drug-like chemicals discovered by bulk methods halted in (or even before) clinical trials, leading to tremendous waste of time and financial costs in drug development.

The heterogeneity of the tumor cells is one of the causes that lead to the tumor relapse and/or resistance to treatment. scRNA-seq can reveal the existence and expression signatures of resistant subgroups for explaining and supporting drug ineffectiveness due to cellular heterogeneity. scRNA-seq is already widely used in the translational research for tumor heterogeneity. However, the power of scRNA-seq analysis has not yet been used for high throughput drug screening.

Cultured primary tumor cells or tumor-derived cell lines have been harnessed as models for drug screening for years due to its convenience, relatively low cost, and physical relevance compared with other models such as genetically engineered mouse or xenografts.

Gene expression profiling, such as microarray or RNA sequencing, has been adopted in drug discovery and drives drug discovery from the commonly used target-based approach into a molecular phenotype-based stage, which does not require known molecular mechanisms.
Using transcriptome changes as a measurement of the response to drug candidates in cell lines accumulates numerous data that constitutes a repertoire of connections, associating drug and disease for drug discovery, as well as comparing disease and drug response signatures for drug repurposing.

Drugs used in cancer therapy are designed to kill the cancer cells. Due to the rapid decline in drug concentration, cells are easily placed in a non-lethal dose environment. When they were in the non-lethal ranges, cells can choose to remain proliferative or become senescent. However, the bulk RNA transcriptome cannot distinguish the developmental directions of the cells.

scRNA-seq can reveal the existence and expression signatures of resistant subgroups for explaining and supporting drug ineffectiveness due to cellular heterogeneity. And effective drugs that are directed against different cell subtypes can be screened or combination of two or more drugs direct against different cell subtypes could be discovered.

During cancer therapy some cancer cells cannot be killed by drugs and become senescent. In one side, scRNA-seq can screen drugs for inducing senescence as a therapeutic strategy with the potential to reduce cytotoxicity and engage the immune system. In another side, the slow development of senescence phenotypes has won challenges for improving medication strategies such as combined medication.

Sequencing at single cell resolution could reveal the heterogeneity of cells which was concealed by averaging the expression of all cells in bulk RNA-seq. The advantages of single-cell gene expression analysis include (1) more accurate characterization of gene expression profiles in individual cells, and (2) identification of cell types, including rare new cell types or subtypes, (3) acquisition of gene expression snapshots during cellular transition from one state to another.

The present invention relates to applying scRNA-seq for drug screening and repurposing in cell lines in a more general and systematical way as a platform for the solution to the drug efficacy-lost problem due to the tumor heterogeneity.

### Summary of the invention

The present invention refers to a method for assaying a cell-based drug screening comprising the steps of:
- culturing a cell population in a culturing environment;
- exposing the cell population to drug substances, wherein defined monitoring points in time and concentration of the drug substances are set;
- generating a single cell library after exposure to drug substances;
- performing single cell RNA sequencing of the single cell libraries on a plurality of genes of the cells from the cell library; and
- performing bioinformatic analysis to identify differentially expressed genes, enriched pathways and developmental trajectories.

The present invention refers to the use of a method for assaying a cell-based drug screening, wherein differentially expressed genes are potential marker genes and are used to screen drugs for cancers.

### Description of the invention

The term "subgroup" herein used refers to cluster single-cell RNA sequencing by bioinformatics algorithms, each of the cluster is a subgroup.

The term "clustering" herein used refers to the classification of data into corresponding classes based on the similarity between single-cell RNA sequencing data. There is a high degree of similarity between the same classes, with the greatest degree of difference between the different classes.

The term "clustering analysis" herein used refers to the process of grouping a collection of single-cell RNA sequencing data into multiple classes consisting of similar gene expression profiles.

The term "bioinformatic analysis" herein used refer to identify differentially expressed genes, clustering analysis, enriched pathways and developmental trajectories and is referred to all the analysis in this patent.

The term "differentially expressed genes (DEGs)" herein used refers to genes whose expressions are significantly different between subgroups of cells.

The term "repurposing" herein used refers to finding a new use for a drug in another disease.

The term "pro-apoptotic genes" herein used refers to a gene that promotes apoptosis of cells.

The term "drug resistant cancer" herein used refers to a cancer that is resistant to drug treatment.

The term "developmental trajectories" herein used refers to simulate the process of cell differentiation according to different cell states.

The term "enriched pathways" herein used refers to enrich the KEGG pathway through differentially expressed genes.

The term "cancer" used herein refers to, but not limited to: biliary tract cancer; bladder cancer; brain cancer including glioblastomas and medulloblastomas; breast cancer; cervical cancer; choriocarcinoma; colon cancer; endometrial cancer; esophageal cancer; gastric cancer; hematological neoplasms including acute lymphocytic and myelogenous leukemia; multiple myeloma; AIDS-associated leukemias and adult T-cell leukemia lymphoma; intraepithelial neoplasms including Bowen's disease and Paget's disease; liver cancer; lung cancer; lymphomas including Hodgkin's disease and lymphocytic lymphomas; neuroblastomas; oral cancer including squamous cell carcinoma; ovarian cancer including those arising from epithelial cells, stromal cells, germ cells and mesenchymal cells; pancreatic cancer; prostate cancer; rectal cancer; sarcomas including leiomyosarcoma, rhabdomyosarcoma, liposarcoma, fibrosarcoma, and osteosarcoma; skin cancer including melanoma, Kaposi's sarcoma, basocellular cancer, and squamous cell cancer; testicular cancer including germinal tumors such as seminoma, non-seminoma (teratomas, choriocarcinomas), stromal tumors, and germ cell tumors; thyroid cancer including thyroid adenocarcinoma and medullar carcinoma; and renal cancer including adenocarcinoma and Wilms tumor, breast, prostate, lung, ovarian, colorectal, and brain cancer.

The term "cancer treatment" as described herein, may include but is not limited to: chemotherapy, radiotherapy, adjuvant therapy, or any combination of the aforementioned methods. Aspects of treatment that may vary include, but are not limited to: dosages, timing of administration, or duration; and may or may not be combined with other treatments, which may also vary in dosage, timing, or duration.

Single-cell RNA sequencing technology for drug screening. The drug-treated single cells are first sequenced, and the sequencing data is then subjected to bioinformatics analysis. scRNA-seq can reveal the existence and expression signatures of resistant subgroups for explaining and supporting drug ineffectiveness due to cellular heterogeneity. And effective drugs that are directed against different cell subtypes can be screened or combination of two or more drugs direct against different cell subtypes could be discovered.

The inventors used single-cell RNA sequencing technology to analyze the changes in the cell population composition and gene expression of tumor cell lines K562 after reaction with the drugs, such as Oxaliplatin and LSD1, in order to select whether these drugs will be used for tumor therapy. The distinguishing and technical feature of the present invention were: (1) there were literatures on the analysis of gene expression of K562 and the drugs Oxaliplatin or LSD1 reactions by RT-qPCR, NGS and flow cytometry, while not by single-cell RNA sequencing; (2) the time and concentrations of the reaction between cell lines and the drugs are the invention of this patent; (3) in this patent, we found about that Oxaliplatin or LSD1 may can be used in leukemia treatment; and (4) the invention relates to applying scRNA-seq for drug screening and repurposing in cell lines.
The inventors found that the time and concentrations of the reaction between cell lines and the drugs can be used in the treatment of leukemia, specifically that Oxaliplatin or LSD1 can be used in the treatment. The invention relates to applying scRNA-seq for drug screening and repurposing in cell lines.

The present invention refers to a method for assaying a cell-based drug screening comprising the steps of:
- culturing a cell population in a culturing environment;
- exposing the cell population to drug substances, wherein defined monitoring points in time and concentration of the drug substances are set;
- generating a single cell library after exposure to drug substances;
- performing single cell RNA sequencing of the single cell libraries on a plurality of genes of the cells from the cell library; and
- performing bioinformatic analysis to identify differentially expressed genes, enriched pathways and developmental trajectories.

In one embodiment, the cell population is selected from primary cells, cell lines, cancer cells.

In one embodiment, the cell population is a K562 cell line. The culturing environment refers to DMEM Cell Culture Media, 10% FBS (Fetal Bovine Serum), and 1% PS (Penicillin and Streptomycin).

In one embodiment, the single cell RNA sequencing of the single cell libraries is performed on a plurality of genes of individually sorted cells from the cell library.

In one embodiment, the drug substances can be any other anti-tumor drugs or drugs with anti-tumor potential or drugs not previously used for anti-tumor purposes.

In one embodiment, the drug substances are Oxaliplatin or lysine-specific histone demethylase 1A (LSD1) inhibitor.

In one embodiment, the monitoring points in time comprises time points between 0 and 6 hrs. In a preferred embodiment, the monitoring points in time comprises time points between 0 and 24 hrs.

In one embodiment, the monitoring points in concentration of the drug substances comprise 50 nM and 5 µM.

In one embodiment, the single cell RNA sequencing comprises analyzing polyA+ mRNA.

In one embodiment, the single cell RNA sequencing comprises analyzing mRNA.

In one embodiment, the differentially expressed genes identified comprise marker genes of cell subtypes that are involved in pathways for drug metabolism, cell growth, reproduction, and differentiation.

In one embodiment, the differentially expressed genes identified comprise BNIP3, BNIP3L, HEMGN and KDM1A.

In one embodiment, the differentially expressed genes identified comprise any genes which is differentially expressed between subgroups of cells. The values are avg_logFC>0, and the proportion of cells expressing this gene in this subgroup is significantly higher than in other subgroups.

In one embodiment, the identified differentially expressed genes for the cell population treated with Oxaliplatin and/or any anti-cancer drug are genes related to RNA transport and spliceosome.

In one embodiment, the single cell RNA sequencing of the single cell libraries is performed on a plurality of genes of individually sorted cells from the cell library.

In one embodiment, the identified differentially expressed genes for the cell population treated with lysine-specific histone demethylase 1A (LSD1) inhibitor are pro-apoptotic genes or genes resistant to lysine-specific histone demethylase 1A (LSD1) inhibitor.

In one embodiment, the identified differentially expressed genes for the cell population treated with lysine-specific histone demethylase 1A (LSD1) inhibitor comprises BNIP3, BNIP3L, HEMGN and KDM1A genes.

The present invention refers to the use of a method for assaying a cell-based drug screening, wherein differentially expressed genes are potential marker genes and are used to screen drugs for cancers.
The use of a method for assaying a cell-based drug screening, wherein differentially expressed genes are potential marker genes and are used to screen drugs for drug-resistant cancers comprising the steps of:
- culturing a cell population in a culturing environment;
- exposing the cell population to drug substances, wherein defined monitoring points in time and concentration of the drug substances are set;
- generating a single cell library after exposure to drug substances;
- performing single cell RNA sequencing of the single cell libraries on a plurality of genes of the cells from the cell library; and
- performing bioinformatic analysis to identify differentially expressed genes, enriched pathways and developmental trajectories.
In one embodiment, the cell population is selected from primary cells, cell lines, cancer cells.

In one embodiment, the cell population is a K562 cell line. The culturing environment refers to DMEM Cell Culture Media, 10% FBS (Fetal Bovine Serum), and 1% PS (Penicillin and Streptomycin).

In one embodiment, the drug substances can be any other anti-tumor drugs or drugs with anti-tumor potential or drugs not previously used for anti-tumor purposes.

In one embodiment, the drug substances are Oxaliplatin or lysine-specific histone demethylase 1A (LSD1) inhibitor.

In one embodiment, the monitoring points in time comprises time points between 0 and 6 hrs. in a preferred embodiment, the monitoring points in time comprises time points between 0 and 24 hrs.

In one embodiment, the single cell RNA sequencing comprises analyzing polyA+ mRNA.

In one embodiment, the single cell RNA sequencing comprises analyzing mRNA.

In one embodiment, the differentially expressed genes identified comprise marker genes of cell subtypes that are involved in pathways for drug metabolism, cell growth, reproduction, and differentiation.

In one embodiment, the differentially expressed genes identified comprise BNIP3, BNIP3L, HEMGN and KDM1A.

In one embodiment, the differentially expressed genes identified comprise any genes which is differentially expressed between a subgroup of cells. In one embodiment, the identified differentially expressed genes for the cell population treated with Oxaliplatin and/or any anti-cancer drug are genes related to RNA transport and spliceosome.

In one embodiment, the identified differentially expressed genes for the cell population treated with lysine-specific histone demethylase 1A (LSD1) inhibitor are pro-apoptotic genes or genes resistant to lysine-specific histone demethylase 1A (LSD1) inhibitor.

In one embodiment, the identified differentially expressed genes for the cell population treated with lysine-specific histone demethylase 1A (LSD1) inhibitor comprises BNIP3, BNIP3L, HEMGN and KDM1A genes.

### EXAMPLES

### Example 1

### Drug treatment, scRNA-seq and analysis of sequencing data

K562 cells are cultured in medium, and then exposed to 50nM of Oxaliplatin. After 0, 2, 6, 24 hr of exposure, cells are washed and resuspended in PBS, samples denoted as K00_0, K02_50, K06_50, K24_50. Single cells were processed through the Single Cell 3' Gel Bead, GEXSCOPE® Microfluidic Chip and Library Kits. Libraries were sequenced on Illumina sequencers.

The changes of composition about cell line populations were shown by UMAP plot. Gene set enrichment analysis (GSEA) was performed on cells.

The total RNA was isolated by AllPrep DNA/RNA Mini Kit (Qiagen Cat No./ID: 80204), and measured by Qubit2.0 Fluorometer (Life Technologies, CA, USA) and NanoPhotometer spectrophotometer (IMPLEN, CA, USA).

Single-cell suspensions with 1×10⁵ cells/mL in concentration in PBS (HyClone) were prepared. Single-cell suspensions were then loaded onto microfluidic devices and scRNA-seq libraries were constructed according to Singleron GEXSCOPE® protocol by GEXSCOPE® Single-Cell RNA Library Kit (Singleron Biotechnologies). Individual libraries were diluted to 4nM and pooled for sequencing. Pools were sequenced on Illumina HiSeq X with 150 bp paired end reads.

Raw reads were processed to generate gene expression profiles using an internal pipeline. Reads with the same cell barcode, UMI and gene were grouped together to calculate the number of UMIs per gene per cell. The UMI count tables of each cellular barcode were used for further analysis. Cell type identification and clustering analysis using Seurat program (http://satijalab.org/seurat/, R package,v.3.0.1).

Statistically significant principle components were selected for UMAP and FindNeighbors analysis in Seurat.
KEGG function enrichment analysis was performed on the gene set using the clusterProfiler software to find biological functions or pathways that are significantly associated with the genes specifically expressed. Differentially expressed genes GSEA analyses by GSEApy(v0.9.12).

### Use scRNA-seq to reveal resistant subgroups

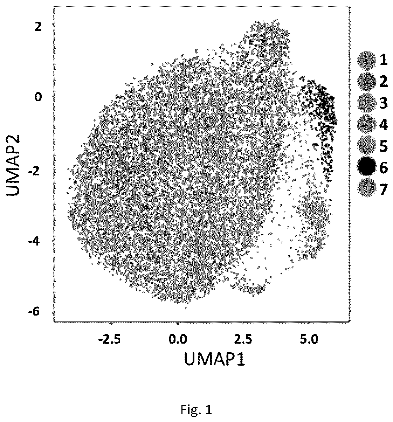

Seurat FindClusters was used to perform unsupervised clustering, and cells were clustered into 7 different subgroups (Figure1). We analyzed the differentially expressed genes (DEGs) of each subgroup as the signature of that group and compared the alteration in subgroups among each time points.

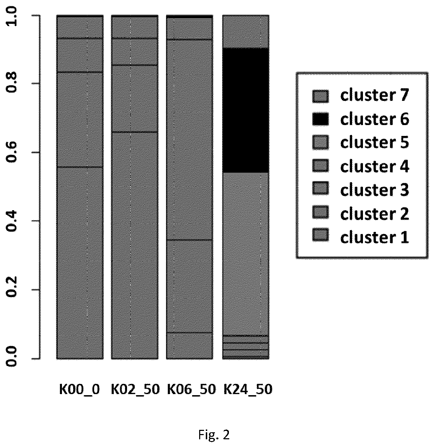

According to the result, the percentage of subgroup 3 increases while that of subgroup 1 decreases, and several new subgroups (subgroups 5-7) emerge during the drug treatment (Figure2).

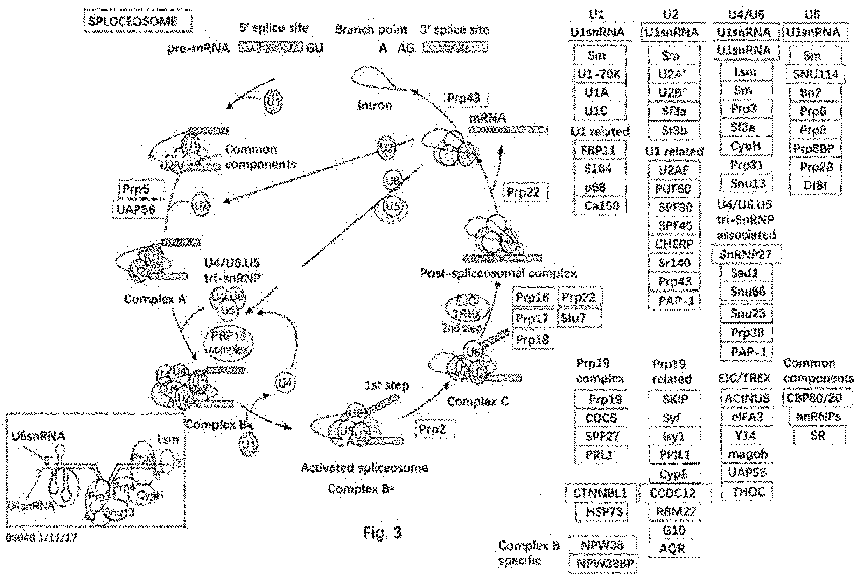

DEGs of subgroup 3 enriches in pathways related to RNA transport and spliceosome, indicating the active transcription which may supporting successive cell dividing and fast growth (Figure 3). As a deduction, we use these DEGs as signatures of subgroups 3, 5, 6 to amend our database, which may assist drug screening for the patients containing similar subgroups of drug resistant cancer cells.

### Example 2

### Drug treatment, scRNA-seq and analysis of sequencing data

K562 cells are cultured in medium, and then exposed to different concentration (0 µM, 5 µM, 20 µM) of Lysine-specific histone demethylase 1A (LSD1) inhibitor for 0, 6, 24 hrs. Cells are washed and resuspended in PBS, samples denoted as LSD1_0_0, LSD1_6_0, LSD1_6_5, LSD1_6_20, LSD1_24_0, LSD1_24_5 and LSD1_24_20. Single cells were processed through the Single Cell 3' Gel Bead, GEXSCOPE® Microfluidic Chip and Library Kits. Libraries were sequenced on Illumina sequencers.

The changes of composition about cell line populations were shown by t-SNE plot. GSEA was performed on cells considering different datasets (Gene Ontology and KEGG Pathway Database) through cluster profiler. Pseudo-time trajectories were identified in each single sample by using Monocle2 with default parameters. Pseudo-time trajectories of all cell lines were performed and the location of each cluster's cells on the trajectory was analyzed.

### Use scRNA-seq to reveal resistant subgroups

Assessing the collective heterogeneity of cells, we observed 5 distinct clusters based on mRNA expression. DEGs of each subgroup were analyzed as the signature of that group and the alterations in subgroups were compared among each drug concentration and time points.
According to the result, as the processing time is gradually extended, the subgroup 3 is increased first and then decreased, and the subgroups 1 and 2 are continuously reduced until it is finally negligible.

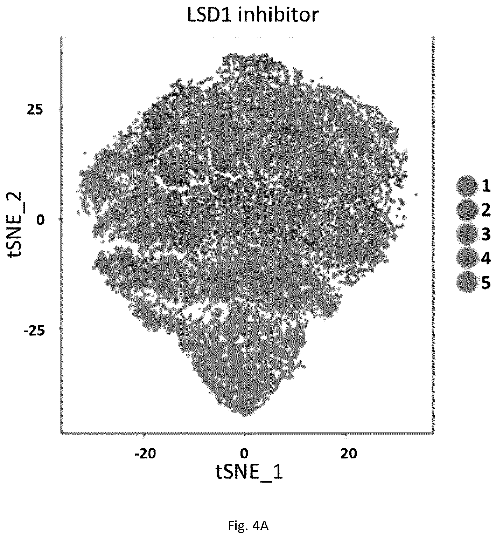

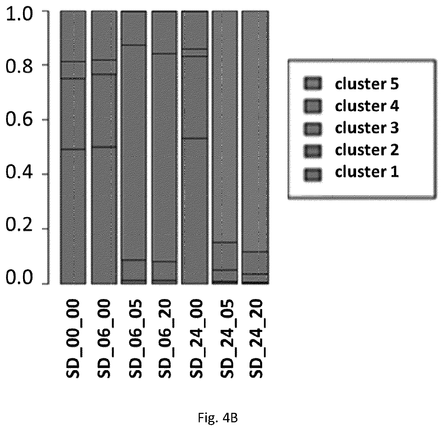

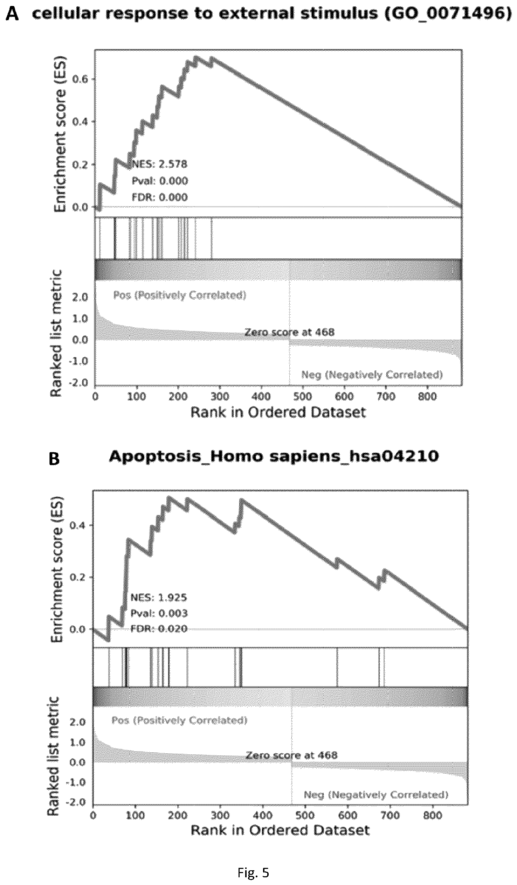

A new subgroup (cluster 5) emerge during the drug treatment 24 h (Figure 4). Subgroup 5 is a cell population that enters the apoptotic pathway, and there is a significant enrichment in cell-stimulated response, secretion, and cell surface-related processes (Figure 5) (ES>0 and Pval<0.05, wherein ES is the enrichment score and Pval is the p value). The vertical coordinates are the enrichment score ES and the association between gene expression and phenotype. The larger the absolute value, the stronger the association. A value greater than 0 indicates a positive correlation, and a value less than 0 indicates a negative correlation.

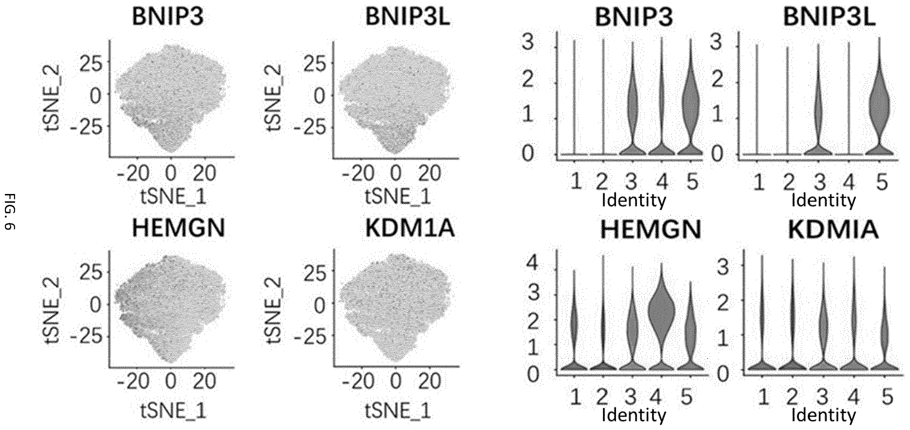

Differential expression in BNIP3, BNIP3L, HEMGN and KDM1A (Figure 6) was found. HEMGN is a prevention of apoptosis genes and has a high expression level in subgroup 4, indicating that the cluster has some resistance to this drug. From the cell subgroups of K562 cell line, some of them showed resistant signature before drug treatment (Figure 4), which highlights the significant heterogeneity even within the population of the cell line.

Both cluster 1 and cluster 2 are drug sensitive cells. However, it can be seen that the expression of HEMGN gene in the cluster 2 is lower, which is easier to be killed by LSD1 inhibitor or it is easier to enter the apoptotic pathway.

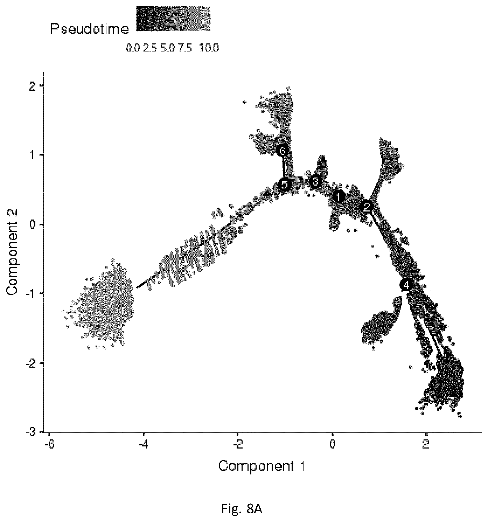

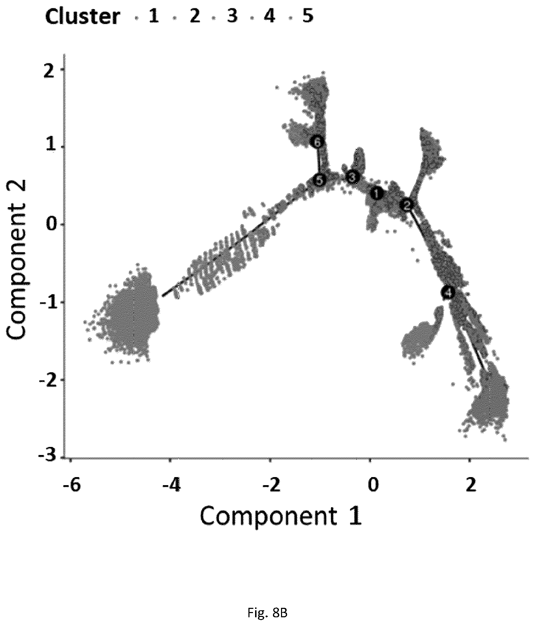

Under the action of LSD1 inhibitor, we observed distinct bifurcated architecture of the cell trajectory, and cluster 1, 2, 3 can be differentiated into two pathways: apoptosis (cluster 5) and resistance (cluster 4) (Figure 8).

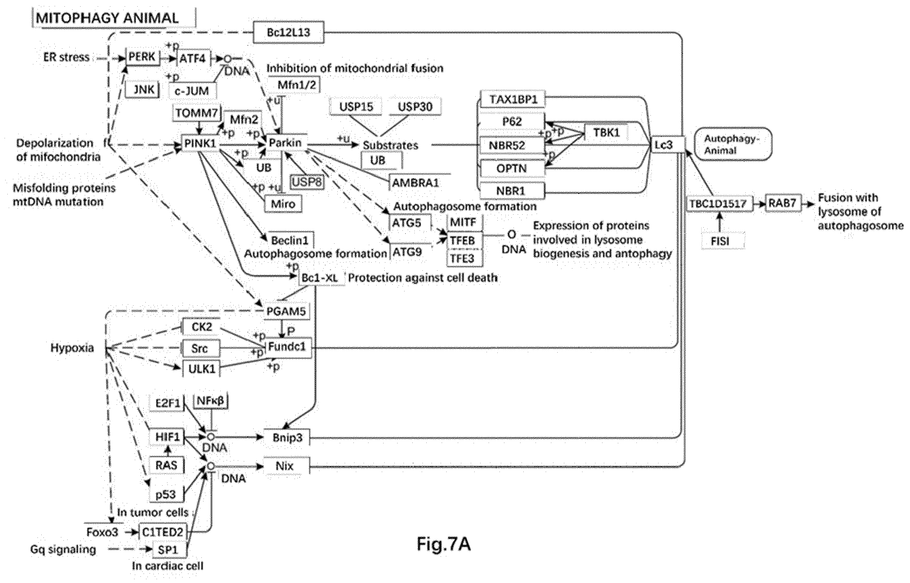

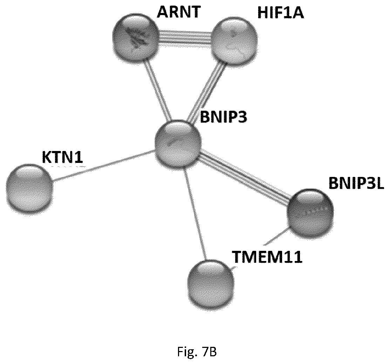

The BNIP3 and BNIP3L genes are pro-apoptotic genes involved in the mitophagy pathway, indicating that cluster 3 is a type of cell population that is transformed into an apoptotic pathway (Figure 7).

These DEGs can be used as potential marker genes to help us screen drugs for drug-resistant cancers with similar cancer cell populations.

### Description of the drawings

Fig1. A UMAP plot of gene expression profiles from multiple samples of cell lines.
   UMAP plot with clusters demarcated by colors demonstrating 7 distinct clusters based on gene expression differences for K562 cell lines which were exposed to 50nM of Oxaliplatin for 0, 2, 6 and 24 hours.
Fig2. Changes in composition of cell line populations at different time points during drug treatment. The Proportion of 7 clusters obtained from Figure 1 in K562 cell lines exposed to 50 nM Oxaliplatin at 0, 2, 6, and 24 hours.
Fig3. KEGG enrichment analysis. KEGG pathway analysis showed that DEGs are involved in the RNA transport and spliceosome, indicating the active transcription which may supporting successive cell dividing and fast growth.
Fig4. A t-SNE plot of gene expression profiles from multiple samples of cell lines (A). t-SNE plot with clusters demarcated by colors demonstrating 5 distinct clusters based on gene expression differences for K562 cell lines which were exposed to 0 µM, 5 µM and 20 µM of Lysine-specific histone demethylase 1A (LSD1) inhibitor for 0, 6 and 24 hours (A). And Changes in composition of 5 clusters in K562 cell line exposed to 0 µM, 5 µM and 20 µM LSD1 at 0, 6, and 24 hours (B).
Fig5. The GSEA analyses of differentially expressed genes by GSEApy (v0.9.12). The cell population entering the apoptotic pathway has a significant positive enrichment in the cell-stimulated response, secretion, and cell surface-related processes (ES>0 and Pval<0.05).
Fig6. Potential novel markers of cell line populations. The feature plot and violin maps indicated the expression of potential new marker genes (BNIP3, BNIP3L, HEMGN and KDM1A) in each cluster.
Fig7. KEGG enrichment analysis. KEGG pathway analysis showed that DEGs are involved in the mitophagy pathway, indicating that cluster 3 is a type of cell population that is transformed into an apoptotic pathway (A) and protein interaction network (B).
Fig8. Cell trajectories defined by single-cell transcriptomes. The unsupervised method of monocle 2 to construct the potential developmental trajectories of cell lines. According to the results of the pseudo-time analysis, it can be found that the differentiation from the right end to the left end of the branch showed the time from front to back (A). The branched trajectory of the cell lines showed the distinct bifurcated architecture (B).

## Claims

1. A method for assaying a cell-based drug screening comprising the steps of:
- culturing a cell population in a culturing environment;
- exposing the cell population to drug substances, wherein defined monitoring points in time and concentration of the drug substances are set;
- generating a single cell library after exposure to drug substances;
- performing single cell RNA sequencing of the single cell libraries on a plurality of genes of the cells from the cell library; and
- performing bioinformatic analysis to identify differentially expressed genes, enriched pathways and developmental trajectories.

2. The method according to claim 1, wherein said cell population is selected from primary cells, cell lines, cancer cells.

3. The method according to claims 1 to 2, wherein the cell population is a K562 cell line.

4. The method according to claims 1 to 3, wherein the drug substances are Oxaliplatin or lysine-specific histone demethylase 1A (LSD1) inhibitor.

5. The method according to claims 1 to 4, wherein the monitoring points in time comprises time points between 0 and 24 hrs.

6. The method according to claims 1 to 5, wherein the single cell RNA sequencing comprises analyzing polyA+ mRNA.

7. The method according to claims 1 to 6, wherein the differentially expressed genes identified comprise marker genes of cell subtypes that are involved in pathways for drug metabolism, cell growth, reproduction, and differentiation.

8. the method according to claims 1 to 7, wherein differentially expressed genes identified comprise BNIP3, BNIP3L, HEMGN and KDM1A.

9. The method according to claims 1 to 8, wherein the identified differentially expressed genes for the cell population treated with Oxaliplatin are genes related to RNA transport and spliceosome.

10. The method according to claims 1 to 9, wherein the identified differentially expressed genes for the cell population treated with lysine-specific histone demethylase 1A (LSD1) inhibitor are pro-apoptotic genes or genes resistant to lysine-specific histone demethylase 1A (LSD1) inhibitor.

11. The method according to claim 1, wherein the identified differentially expressed genes for the cell population treated with lysine-specific histone demethylase 1A (LSD1) inhibitor comprises BNIP3, BNIP3L, HEMGN and KDM1A genes.

12. Use of a method for assaying a cell-based drug screening according to claims 1 to 10, wherein differentially expressed genes are potential marker genes and are used to screen drugs for drug-resistant cancers.
